Europäisches Patentamt

European Patent Office

Office européen des brevets

(11) Veröffentlichungsnummer : **0 120 220**
**B1**

(12) **EUROPÄISCHE PATENTSCHRIFT**

(45) Veröffentlichungstag der Patentschrift :
23.03.88

(21) Anmeldenummer : 84100965.7

(22) Anmeldetag : 31.01.84

(51) Int. Cl.⁴ : **C 12 Q    1/54**

(54) Verfahren und Reagenz zur Glucosebestimmung im hämolysierten Blut.

(30) Priorität : 31.01.83 DE 3303098

(43) Veröffentlichungstag der Anmeldung :
03.10.84 Patentblatt 84/40

(45) Bekanntmachung des Hinweises auf die Patenterteilung : 23.03.88 Patentblatt 88/12

(84) Benannte Vertragsstaaten :
AT BE CH DE FR GB IT LI LU NL SE

(56) Entgegenhaltungen :
EP-A- 0 012 184
DE-A- 2 816 229
GB-A- 1 163 409
US-A- 4 271 264
CHEMICAL ABSTRACTS, Band 69, 1968, Seite 9703,
Nr. 103698n, Columbus, Ohio, US; J.E. MIDDLETON:
"Preparation and investigation of a stabilized glucose oxidase-peroxidase reagent for estimating glucose, using o-tolidine with an alkylaryl sulfonate and
polyethylene glycol"

(73) Patentinhaber : Boehringer Mannheim GmbH
Sandhofer Strasse 116
D-6800 Mannheim 31 (DE)

(72) Erfinder : Draeger, Brigitte, Dr.
Hofmairstrasse 15
D-8132 Tutzing (DE)
Erfinder : Ziegenhorn, Joachim, Dr.
Ina-Seidel-Weg 1
D-8130 Starnberg (DE)

(74) Vertreter : Weickmann, Heinrich, Dipl.-Ing. et al
Patentanwälte Dipl.-Ing. H.Weickmann Dipl.-Phys.Dr.
K.Fincke Dipl.-Ing. F.A.Weickmann Dipl.-Chem. B.
Huber Dr.-Ing. H. Liska Dipl.-Phys.Dr. J. Prechtel
Postfach 860820
D-8000 München 86 (DE)

EP 0 120 220 B1

Anmerkung : Innerhalb von neun Monaten nach der Bekanntmachung des Hinweises auf die Erteilung des
europäischen Patents im Europäischen Patentblatt kann jedermann beim Europäischen Patentamt gegen das erteilte
europäische Patent Einspruch einlegen. Der Einspruch ist schriftlich einzureichen und zu begründen. Er gilt erst als
eingelegt, wenn die Einspruchsgebühr entrichtet worden ist (Art. 99(1) Europäisches Patentübereinkommen).

## Beschreibung

Die Erfindung betrifft ein Verfahren und ein Reagenz zur Bestimmung der Blutglucose im hämolysierten Vollblut nach dem Hexokinase/Glucose-6-Phosphat-Dehydrogenase-Verfahren.

Die Bestimmung des Blutglucosespiegels gehört zu den häufigsten klinisch-chemischen Analysen im Laboratorium. Sie wird sowohl in großer Anzahl in der Routine als auch in der Notfallanalytik durchgeführt.

Vor allem für die Blutglucosekontrolle bei Diabetikern werden für den Glucosenachweis kleine Probenvolumina (Entnahme von Kapillarblut aus der Fingerbeere), Stabilität der Probe über einen längeren Zeitraum (Versand der Probe) und einfache Durchführbarkeit der Bestimmung bei hoher Präzision und Richtigkeit gefordert.

Zur Bestimmung der D-Glucose-Konzentration in Körperflüssigkeiten wird die Hexokinase-, Glucose-6-Phosphatdehydrogenase-Methode international als Referenzmethode angesehen. Bei dieser Methode wird D-Glucose mit ATP in Gegenwart von Hexokinase (HK) in Glucose-6-phosphat überführt und letzteres mit Glucose-6-Phosphat-dehydrogenase (G6PDH) und NADP zu Gluconat-6-Phosphat und NADPH + $H^+$ umgesetzt.

Als Probe wird normalerweise enteiweißtes Vollblut oder Serum bzw. Plasma eingesetzt. Dies erfordert eine zeit- und arbeitsaufwendige Probenvorbehandlung.

Wesentlich einfacher wäre es, die im Blut vorhandenen Zellen (Erythrozyten) durch geeignete Zusätze aufzulösen (Hämolyse) und in der so erhaltenen homogenen Lösung Glucose zu bestimmen.

Voraussetzung für eine richtige Glucosebestimmung im Hämolysat ist jedoch, daß die in den menschlichen Erythrocyten vorhandenen Glucose umsetzenden Enzyme der Glykolyse und des Pentosephosphatzyklus, die bei der Hämolyse freigesetzt werden, inhibiert werden.

In der Literatur sind verschiedene Inhibitoren für die Erythrocytenenzyme beschrieben (z. B. Fluoride, N-Alkylmaleinimide, Halogenacetate etc.), deren Nachteil jedoch darin besteht, daß sie die Glykolyse im Hämolysat nicht vollständig unterbinden oder daß die Haltbarkeit der Inhibitoren in Lösung ungenügend ist (z. B. Maleinimid, Halogenacetate).

Beispielsweise ist eine wichtige Voraussetzung für eine Bestimmung der Glucose im Hämolysat mit Hilfe der Hexokinasemethode als Nachweissystem die vollständige Hemmung der in den Erythrocyten enthaltenen Gluconat-6-Phosphatdehydrogenase, da dieses Enzym Gluconat-6-phosphat, das beim Glucosenachweis entsteht, unter NADPH-Bildung weiter umsetzt, was zu hohe Glucosewerte vortäuscht.

Ein in neuerer Zeit beschriebenes Hämolysemittel zur Bestimmung der Glucose im Blut, bestehend aus einer gepufferten EDTA-Detergenslösung, ist zwar stabil und hemmt den Glucoseabbau im Hämolysat, inhibiert aber nicht ausreichend die Gluconat-6-Phosphatdehydrogenase, was zu einer Schleichreaktion im Testsystem führt.

Keine Beeinflussung der Glucosebestimmung nach der Hexokinasemethode zeigt ein Hämolysemittel bestehend aus Maleinimid und Digitonin, das jedoch zwei andere Nachteile aufweist : begrenzte Stabilität des Hämolysemittels und Eintrübung der Hämolysate bei längerer Lagerung als 3 Tage bei Raumtemperatur.

Der Erfindung liegt daher die Aufgabe zugrunde, ein Verfahren zur Durchführung der Blutglucosebestimmung im hämolysierten Blut nach der Hexokinase/Glucose-6-Phosphatdehydrogenase-Methode zu schaffen, welches die Nachteile der bekannten Methoden nicht aufweist und insbesondere die aus den Erythrocyten freigesetzten Enzyme der Glycolyse und des Pentosephosphatzyklus vollständig inhibiert, ein stabiles Hämolysemittel liefert und weder eine Eintrübung der Hämolysate bei Lagerung noch eine Instabilität der Glucose im Hämolysat hervorruft.

Gelöst wird diese Aufgabe erfindungsgemäß durch ein Verfahren zur Bestimmung von D-Glucose in Körperflüssigkeiten durch Umsetzung mit ATP in Gegenwart von Hexokinase, Glucose-6-Phosphatdehydrogenase, NADP, $Mg^{++}$-Ionen und Puffer und Messung von gebildetem NADPH, welches dadurch gekennzeichnet ist, daß man Vollblut mit 0,01 bis 0,5 % Gewicht/Volumen eines Alkylsulfats oder -sulfonats oder Alkylarylsulfonats, wobei die Alkylgruppen 8 bis 32 C-Atome enthalten und eine oder mehrere Hydroxyalkylaminogruppen als Substituenten aufweisen können oder/und durch ein oder mehrere Äthersauerstoffatome unterbrochen sein können und die Alkyl-Aryl-Gruppen 8 bis 18 C-Atome im Alkylrest aufweisen und gegebenenfalls einem Konservierungsmittel versetzt und dann direkt die genannten weiteren Reagenzien zusetzt.

Bei der Durchführung des erfindungsgemäßen Verfahrens gibt man in der Regel eine kleine Menge des Vollbluts zu einer Lösung, die die angegebene Konzentration an Sulfat oder Sulfonat enthält, beispielsweise im Verhältnis Blut zu Hämolysiermittellösung von 1 bis 4 zu 100.

Es hat sich überraschenderweise gezeigt, daß die genannten Alkylsulfate, Alkylsulfonate und Alkylarylsulfonate eine äußerst rasche Hämolyse der Erythrocyten im Blut bewirken und die dabei freigesetzten Enzyme so vollständig inhibieren, daß keine Störung der HK/G6PDH-Methode durch sie hervorgerufen wird, andererseits aber auch keinerlei Störung dieser Methode selbst bzw. Hemmung der eingesetzten Enzyme hervorgerufen wird. Das erfindungsgemäße Verfahren liefert ein stabiles Meßsignal (Endpunktbestimmer), welches bei dem bekannten Hämolysereagenz aus EDTA und Polyoxyäthylen-10-Alkylphenoläther nicht erhalten wird.

Es war nicht vonnersehbar, daß mit dem erfindungsgemäßen Verfahren die eingangs genannte Aufgabe gelöst werden kann, da es aus Life Sciences Vol. 31 pp 463-470 (1982) bekannt ist, daß z. B. Dodezylsulfat die G6PDH gegenüber Glucose-6-phosphat kompetitiv hemmt. Man mußte daher erwarten, daß in Gegenwart der erfindungsgemäß verwendeten Alkylsulfate, -sulfonate oder/und Alkylarylsulfonate die HK/G6PDH-Methode nicht durchführbar sein würde.

Bei den erfindungsgemäß verwendeten Sulfaten und Sulfonaten liegt die Länge der Alkylgruppen, wenn sie nicht von Äthersauerstoffatomen unterbrochen sind, vorzugsweise zwischen 10 und 18 C-Atomen, wenn sie eine Ätherbrücke enthalten, zwischen 20 und 28 C-Atomen. Als Arylgruppe wird die Phenylgruppe bevorzugt. Unter den Hydroxyalkylaminogruppen werden die Mono-, Di- und Triäthanolamingruppen bevorzugt, der Alkanolrest in der Amingruppe kann jedoch 1 bis 4 Kohlenstoffatome enthalten.

Typische Beispiele für erfindungsgemäß geeignete Sulfate und Sulfonate sind Decylsulfat, Undecan-1-Sulfonat, Tetradecylsulfat, Laurylmyristyläthersulfat, Monoäthanolaminlaurylsulfat, Triäthanolaminlaurylsulfat, Dodecylphenylsulfonat und Tetrapropylenbenzolsulfonat.

Die erfindungsgemäß verwendeten organischen Sulfate bzw. Sulfonate können in reiner Form eingesetzt werden. Es lassen sich jedoch auch Gemische verwenden, so wie sie handelsüblich erhältlich sind. Als besonders geeignet erwiesen sich die im Handel als Dodecylsulfat bezeichneten Mischungen, die neben Dodecylsulfat selbst auch einen gewissen Anteil an längere und kürzere Alkylketten aufweisenden Sulfaten tragen.

Die erfindungsgemäß verwendeten organischen Sulfate und Sulfonate werden vorzugsweise in Form ihrer Natrium-, Lithium- und Ammoniumsalze verwendet. Es sind jedoch auch die Salze anderer nicht störender Kationen anwendbar.

Die Konzentration des erfindungsgemäß verwendeten Sulfats oder Sulfonats muß im Bereich von 0,01 bis 0,5 % Gewicht pro Volumen des Hämolysats liegen. Bei geringerer Konzentration ist die Hemmung der störenden Enzyme nicht vollständig, bei höherem Zusatz werden auch die für die Bestimmung benötigten Enzyme gehemmt.

In der Regel verwendet man eine wässrige Lösung mit dem angegebenen Gehalt an Sulfat oder Sulfonat, der man 10 bis 30 µl, vorzugsweise 15 bis 25 µl Vollblut je ml Reagenzlösung zusetzt.

Zweckmäßig wird außerdem ein Konservierungsmittel zugesetzt welches sowohl zur Konservierung des Hämolysemittels als auch zur Konservierung des Hämolysats selbst dient und es so möglich macht, das Verfahren nach Herstellung des Hämolysats längere Zeit zu unterbrechen, ohne daß hierdurch die Analysenergebnisse beeinflußt werden.

Bevorzugtes Konservierungsmittel sind die Alkaliazide, insbesondere Natriumazid. Jedoch erwiesen sich auch andere übliche Konservierungsmittel wie Thiozid, Chlorhexidin und Imidazolinharnstoff als geeignet, worunter zu verstehen ist, daß sie den Test nicht stören. Die genannten Konservierungsmittel werden dabei in den für sie üblichen Konzentrationen verwendet, die beispielsweise bei den Aziden etwa 0,1 mg/ml, bei Thiozid etwa 0,2 mg/ml, bei Chlorhexidin etwa 0,25 mg/ml und bei Imidazolinharnstoff etwa 10 mg/ml betragen.

Ein weiterer Gegenstand der Erfindung ist ein Reagenz zur Durchführung des erfindungsgemäßen Verfahrens, welches dadurch gekennzeichnet ist, daß es Alkylsulfat oder -sulfonat oder Alkylarylsulfonat, wobei die Alkylgruppen 8 bis 32 C-Atome enthalten und eine oder mehrere Hydroxyalkylaminogruppen als Substituenten aufweisen können oder/und durch ein oder mehrere Äthersauerstoffatome unterbrochen sein können und die Alkyl-Aryl-Gruppen 8 bis 18 C-Atome im Alkylrest aufweisen und ein Konservierungsmittel und gegebenenfalls Puffer pH 6 bis 9 enthält. Das Sulfat oder Sulfonat liegt zweckmäßig in Form des Natrium-, Lithium- oder Ammoniumsalzes vor. Als Konservierungsmittel werden die oben erwähnten bevorzugt, besonders bevorzugt werden die Alkaliazide, insbesondere Natriumazid.

Das Verhältnis von Sulfat bzw. Sulfonat zu Konservierungsmittel hängt im wesentlichen von der Art des Konservierungsmittels ab, die wiederum die Konzentration in der gebrauchsfertigen Hämolysereagenzlösung bedingt. Normalerweise wird die Menge des Konservierungsmittels so bemessen, daß sich in der gebrauchsfertigen Hämolysereagenzlösung die Konzentrationen ergeben, die für das Konservierungsmittel normalerweise empfohlen werden. Übliche Werte für die genannten Konservierungsmittel sind oben angegeben. Für die Zusammensetzung Dodecylsulfat/Natriumazid ergibt sich daraus ein Gewichtsverhältnis von 0,3 bis 5 Gewichtsteile Dodecylsulfat auf ein Gewichtsteil Natriumazid.

Das erfindungsgemäße Verfahren ergibt im Vergleich zur Referenzmethode bei Durchführung im enteiweißten Blut ausgezeichnete Übereinstimmung, wie die nachstehende Tabelle zeigt :

| Referenzmethode (Probe : enteiweißtes Blut) | Hämolysatmethode gemäß Erfindung |
|---|---|
| 155,3 mg/dl | 155,2 mg/dl |
| 151,3 mg/dl | 150,6 mg/dl |
| 79,3 mg/dl | 77,6 mg/dl |
| 105,4 mg/dl | 107,5 mg/dl |

Das erfindungsgemäße Verfahren und Reagenz erfüllt alle Forderungen, die an eine praktikable Hämolysatmethode gestellt werden müssen :

a) Kleine Blutvolumina (Venenblut, Kapillarblut) : 20 µl/1 ml Hämolysereagenz bzw. 10 µl/0,5 ml Hämolysereagenz.

b) Keine Teststörung in der Hexokinasemethode : keine Schleichreaktion, gute Übereinstimmung zur Referenzmethode (enteiweißtes Blut als Probe und die Hexokinasemethode zum Glucosenachweis).

c) Sehr gute Stabilität der Glucose im Hämolysat (30 Tage Raumtemperatur), d. h. sehr gute Hemmung der im Hämolysat vorhandenen Glucose umsetzenden Enzyme.

d) Unbegrenzte Haltbarkeit des Hämolysereagenzes.

e) Einfache Herstellung des Reagenzes. Auflösen des Alkylsulfats oder -sulfonats, z. B. von Dodecylsulfat in Wasser ; Zusatz des konservierenden Mittels.

f) Sehr gute hämolysierende Eigenschaften. Die Hämolyse erfolgt innerhalb weniger Sekunden. Im Hämolysat bilden sich auch über lange Zeiträume (30 Tage Raumtemperatur) keine Niederschläge. Die folgenden Beispiele erläutern die Erfindung weiter.

## Beispiel 1

A) Herstellung des Hämolysereagenzes

1,8 g Ammoniumdodecylsulfat und 1 g Natriumazid werden in 1 l Wasser gelöst.

B) Testdurchführung

a) Herstellung des Hämolysats

20 µl Blut werden mit 1 ml des Hämolysereagenzes versetzt. Zur raschen Hämolyse wird das Gemisch kurz geschüttelt.

b) Messung der Glucose im Hämolysat

1. Endpunktbestimmung (manuelle Testdurchführung)

500 µl des Hämolysats werden mit 2 ml folgenden Reagenzes versetzt :
35 mmol/l Phosphatpuffer, pH = 7,7 ;
2 mmol Magnesiumsulfat ; 0,65 mmol NADP ; 0,65 mmol ATP.
Die Extinktion des Gemisches aus Hämolysat und Reagenz wird bei einer Wellenlänge von 365 nm gemessen. Durch Zugabe von 20 µl Enzymlösung, die 125 KU/l Glucose-6-Phosphat-Dehydrogenase und 110 KU/l Hexokinase enthält, wird die Reaktion gestartet. Nach 5 Minuten ist die Reaktion beendet. Aus der Extinktionsdifferenz vor und nach Zugabe der Enzymlösung läßt sich die Glucosekonzentration des Blutes berechnen.
mg/dl Glucose = $1323,4 \times \Delta E_{365}$ nm.

## Beispiel 2

Kinetische Bestimmung (Analysenautomat : Eppendorf ACP 5040)

Reagenz zur Glucosebestimmung

70 mmol/l Phosphatpuffer (pH = 7,7), 4 mmol/l Magnesiumsulfat, 1,3 mmol/l NADP, 5 mmol/l ATP.
Enzymlösung : 12,5 KU G-6-PDH, 11 KU Hexokinase.

Testdurchführung

250 µl Reagenz werden mit 50 µl eines gemäß Beispiel 1 B) erhaltenen Hämolysats gemischt und die Reaktion mit 25 µl der im Beispiel 1 angegebenen Enzymlösung gestartet. 30 Sekunden nach Reaktionsstart wird die Kinetik über 6,7 Sekunden vermessen.
Die Eichung erfolgt über einen Glucosestandard. Der Ausdruck der Werte erfolgt in mg/dl Glucose.

## Beispiele 3 bis 13

Es wurde, wie im Beispiel 1 beschrieben vorgegangen, jedoch wurden anstelle des Dodecylsulfats die in der nachstehenden Tabelle aufgeführten Sulfate bzw. Sulfonate verwendet. Die Tabelle zeigt weiter die jeweils angewendeten Konzentrationen im Hämolysiermittel, wobei sich diese Konzentrationsangabe auf das Handelsprodukt bezieht, sowie die über 30 Minuten verfolgte Extinktionsänderung und schließlich die Wiederfindung der eingesetzten Glucose in %.

4

| Beispiel | Sulfat oder Sulfonat (Warenzeichen als solche anerkannt) | Konzentration im Hämolysier- mittel | Extinktionsänderung 5.-35. Minute nach Reaktionsstart (Schleichreaktion) | Wiederfindung Glucose |
|---|---|---|---|---|
| 3 | Decylsulfat | 0,25 % | 4 mE[1] | 96 % |
| 4 | Undecan-1-Sulfonat | 0,25 % | − 2 mE[1] | 96 % |
| 5 | Tetradecylsulfat* | 0,25 % | 2 mE[1] | 100 % |
| 6 | Texapon F (Gemisch aus Fettalkoholsul- faten und Alkylarylsulfonaten ; 45 %) | 0,2 % | 2 mE[1] | 99 % |
| 7 | Merpisap DP 82 (Tetrapropylenbenzolsulfonat ; 83 %) | 0,1 % | 2 mE[1] | 103 % |
| 8 | Reworyl NKS 50 (Dodecylphenylsulfonat ; 50 %) | 0,15 % | 1 mE[1] | 100 % |
| 9 | Hostapur AT (Alkansulfonate) | 0,15 % | 2 mE[1] | 100 % |
| 10 | Texapon K 14 S Spezial (Laurylmyristyläthersulfat ; 30 %) | 1 % | 1 mE[2] | 99 % |
| 11 | Texapon MLS (Monoäthanolaminlaurylsulfat ; 28 %) | 0,05 % | 2 mE[2] | 98 % |
| 12 | Texapon ASV (Mischung spezieller Alkylsul- fate ; 28 %) | 0,5 % | − 2 mE[2] | 106 % |
| 13 | Texapon TH (Triäthanolaminlaurylsulfat ; 47-48 %) | 0,5 % | − 3 mE[2] | 97 % |

Angaben der Zusammensetzung und Konzentration sind entnommen aus : Emulsifiers and Detergens, International Edition 1981.

[1]) 20 µl Blut/ml Hämolysereagenz, Messung bei 366 nm
[2]) 10 µl Blut/ml Hämolysereagenz, Messung bei 334 nm
* Um die Substanz in Lösung zu bringen, wurden dem Reagenz 0,5 % Isooctylphenaläther zugesetzt.

**Patentansprüche**

1. Verfahren zur Bestimmung von D-Glucose in Körperflüssigkeit durch Umsetzung mit ATP in Gegenwart von Hexokinase, Glucose-6-Phosphat-dehydrogenase, NADP, $Mg^{++}$-Ionen und Puffer und Messung von gebildetem NADPH, dadurch gekennzeichnet, daß man Vollblut mit 0,01 bis 0,5 % Gewicht/Volumen eines Alkylsulfats oder -sulfonats oder Alkylarylsulfonats, wobei die Alkylgruppe 8 bis 32 C-Atome enthalten und eine oder mehrere Hydroxyalkylaminogruppen als Substituenten aufweisen können oder/und durch ein oder mehrere Äthersauerstoffatome unterbrochen sein können und die Alkyl-Aryl-Gruppen 8 bis 18 C-Atome im Alkylrest aufweisen und gegebenenfalls einem Konservierungsmittel versetzt und dann direkt die genannten weiteren Reagenzien zusetzt.

2. Verfahren nach Anspruch 1, dadurch gekennzeichnet, daß man Dodecylsulfat verwendet.

3. Verfahren nach Anspruch 1 oder 2, dadurch gekennzeichnet, daß man als Konservierungsmittel Alkaliazid, Thiozid, Chlorhexidin oder/und Imidazolinharnstoff verwendet.

4. Reagenz zur Durchführung des Verfahrens nach Anspruch 1 bis 3, dadurch gekennzeichnet, daß es Alkylsulfat oder -sulfonat oder Alkylarylsulfonat, wobei die Alkylgruppen 8 bis 32 C-Atome enthalten und eine oder mehrere Hydroxyalkylaminogruppen als Substituenten aufweisen können oder/und durch ein oder mehrere Äthersauerstoffatome unterbrochen sein können und die Alkyl-Aryl-Gruppen 8 bis 18 C-Atome im Alkylrest aufweisen und ein Konservierungsmittel enthält.

5. Reagenz nach Anspruch 4, dadurch gekennzeichnet, daß es aus Dodecylsulfat und einem Konservierungsmittel aus der Gruppe Alkaliazid, Thiozid, Chlorhexidin oder/und Imidazolinharnstoff besteht.

6. Reagenz nach Anspruch 5, dadurch gekennzeichnet, daß es Dodecylsulfat und Natriumazid im Gewichtsverhältnis 0,3 bis 5 : 1 enthält.

## Claims

1. Process for the determination of D-glucose in body fluids by reaction with ATP in the presence of hexokinase, glucose-6-phosphate dehydrogenase, NADP, $Mg^{++}$ ions and buffer and measurement of the NADPH formed, characterised in that one mixes whole blood with 0.01 to 0.5 % weight/volume of an alkyl sulphate or sulphonate or of alkyl-aryl sulphonate, whereby the alkyl groups contain 8 to 32 C-atoms and can contain one or more hydroxyalkylamino groups as substituents and/or can be interrupted by one or more ether oxygen atoms and the alkyl-aryl groups contain 8 to 18 C-atoms in the alkyl radical, and possibly a preserving agent, and then adds the said further reagents directly.

2. Process according to claim 1, characterised in that one uses dodecyl sulphate.

3. Process according to claim 1 or 2, characterised in that, as preserving agent, one uses alkali metal azide, thiozide, chlorhexidine and/or imidazoline-urea.

4. Reagent for the carrying out of the process according to claim 1 to 3, characterised in that it contains alkyl sulphate or sulphonate or alkyl-aryl sulphonate, whereby the alkyl groups contain 8 to 32 C-atoms and can contain one or more hydroxyalkylamino groups as substituents and/or can be interrupted by one or more ether oxygen atoms and the alkyl-aryl groups contain 8 to 18 C-atoms in the alkyl radical, and a preserving agent.

5. Reagent according to claim 4, characterised in that it consists of dodecyl sulphate and a preserving agent of the group alkali metal azides, thiozide, chlorhexidine and/or imidazoline-urea.

6. Reagent according to claim 5, characterised in that it contains dodecyl sulphate and sodium azide in the weight ratio of 0.3 to 5 : 1.

## Revendications

1. Procédé de détermination du D-glucose dans les liquides corporels par réaction avec l'ATP en présence d'hexokinase, de glucose-6-phosphate-déshydrogénase, de NADP, d'ions $Mg^{++}$ et de tampon et mesure du NADPH formé, caractérisé en ce qu'on additionne le sang total de 0,01 à 0,5 % en poids/volume d'un alkylsulfate ou -sulfonate ou d'un alkylarylsulfonate, les groupes alkyles contenant 8 à 32 atomes de C, et pouvant présenter un ou plusieurs groupes hydroxyalkylamino comme substituants ou/et être interrompus par un ou plusieurs atomes d'oxygène d'éthers, et les groupes alkylaryles présentant 8 à 18 atomes de C dans le radical alkyle, et le cas échéant d'un conservateur, puis en ce qu'on ajoute directement les autres réactifs indiqués.

2. Procédé suivant la revendication 1, caractérisé en ce qu'on utilise du dodécylsulfate.

3. Procédé suivant la revendication 1 ou 2, caractérisé en ce qu'on utilise comme conservateur un azide alcalin, le thiozide, la chlorhexidine ou/et l'imidazoline-urée.

4. Réactif pour l'exécution du procédé suivant les revendications 1 à 3, caractérisé en ce qu'il contient de l'alkylsulfate ou -sulfonate ou de l'alkylarylsulfonate, les groupes alkyles contenant 8 à 32 atomes de C et pouvant présenter un ou plusieurs groupes hydroxyalkylamino comme substituants, ou/et pouvant être interrompus par un ou plusieurs atomes d'oxygène d'éthers, et les groupes alkylaryles présentant 8 à 18 atomes de C dans le radical alkyle, et un conservateur.

5. Réactif suivant la revendication 4, caractérisé en ce qu'il se compose de dodécylsulfate et d'un conservateur du groupe des azides alcalins, du thiozide, de la chlorhexidine ou/et de l'imidazoline-urée.

6. Réactif suivant la revendication 5, caractérisé en ce qu'il contient du dodécylsulfate et de l'azide de sodium dans le rapport pondéral 0,3 à 5 : 1.